# EUROPEAN PATENT APPLICATION

(11) **EP 2 597 162 A1**
(43) Date of publication of application: **29.05.2013**
(21) Application number: 12199212.7
(22) Date of filing: 30.04.2004
(51) Int. Cl.: C12Q 1/68, C12Q 1/70

(54) **Nucleic acid detection**

(30) Priority: 02.05.2003 GB 0310181
(62) Divisional of application: 04730463.9
(71) Applicant: Hai Kang Life Corporation Limited, Mongkok Kowloon, Hong Kong (CN)
(72) Inventor: Yu, Cheung, Hoi, Hong Kong (CN); Lau, Lok, Ting, Kowloon (CN)
(74) Representative: Arends, William Gerrit

(57) **Abstract**

The present invention relates to a process for the detection of nucleic acids from biological or environmental samples. The technique involves the amplification of the nucleic acid of interest, after conversion to cDNA if necessary, after which the amplified nucleic acid is used as the template for further amplification by the real-time (RT-PCR) technique. This combination of amplification procedures increases the sensitivity and specificity of the amplification compared with conventional amplification techniques. The invention also relates to primers for the use in this method and a diagnostic test kit.

## Description

### Field of the invention

This invention relates to a method for the detection of nucleic acids from biological or environmental samples. The invention also relates to a diagnostic test kit for detecting nucleic acids and primers and probes for use in the detection method.

### Background of the invention

Severe Acute Respiratory Syndrome (SARS) is a newly identified form of atypical pneumonia in humans (Drosten et al., "Identification of a novel coronavirus in patients with severe acute respiratory syndrome" The New England Journal of Medicine, (2003) 348:1967-11976, published on 15 May 2003), It is caused by a new strain of coronavirus, known as the SARS coronavirus or Urbani SARS-associated coronavirus. The virus is efficiently spread by acrosol and person-to-person contact has been demonstrated. A diagnosis of suspected or probable SARS is made by following WHO diagnostic criteria as specified in Case Definitions for Surveillance of Severe Acute Respiratory Syndrome (SARS) (World Health Organization, as revised on 30 April 2003), However, a positive diagnosis of SARS cannot be made without identification of the aetiological agent, either by demonstration of the presence of antibodies to the novel coronavirus or by demonstration of the presence of coronavirus nucleic acid in patient samples. The course of the disease can lead to irreversible lung damage and a mortality of 5-10% in confirmed SARS affected patients has been reported. To limit the severity of the disease and to control the spread of the disease, it is important to identify infected patients as soon as possible in order to initiate prompt treatment.

The most commonly used methods to identify viral infections are identification of antibodies in patient serum samples by standard serological methods, including but not limited to agar gel immunodiffusion (AGID) or enzyme-linked immunosorbent assay (ELISA). These methods are limited as it may take seven to twenty-one days after infection for antibodies to be produced in sufficient concentration to enable accurate and sensitive detection.

Culturing the virus in susceptible cells in vitro is also a well-established method for detecting virus. However, the coronavirus associated with SARS is a novel entity and optimum growth conditions have not been described. In addition, growth of the virus requires a high level of biosecurity, as the product of the assay is intact infections virus. Highly skilled staff, equipment and facilities are therefore required, which limits the suitability of this technique for routine diagnostic use. In addition, the growth of the virus may be too slow to enable detection in sufficient time to initiate prompt treatment.

Molecular method, based on the amplification and detection of the virus genetic material, have previously been described for many pathogenic viruses and are applicable to the detection of the SARS coronavirus. Nucleic acid amplification methods in general are rapid, highly specific and sensitive.

Many different types of nucleic acid amplification process have been described including polymerase chain reaction (PCR) followed by agarose gel electrophoresis, nested PCR, real-time Taqman^{®} PCR, molecular beacon PCR, nucleic acid sequence-based amplification (NASBA), isothermal and chimeric primer-initiated amplification of nucleic acids (ICAN), and so forth.

PCR followed by gel by electrophoresis (also known as conventional PCR) is a standard technique that was first described in 1985. It has since become a routine laboratory procedure. It is however, relatively slow and labour intensive. In addition, compared with more recent developments conventional PCR has a lower sensitivity,

Nested PCR followed by gel electrophoresis is a technique that increases the sensitivity of conventional PCR. Nested PCR involves the first amplification of a specific genetic sequence using a particular pair of DNA oligonucleotide primers (outer primers). The product of this amplification is used as the template for a second round of amplification in which another pair of primers (inner primers) are selected from a region within the first amplification product. The second amplification product is thus shorter than the first amplification product. This method is very laborious as it involves an additional amplification step, Consequently, this method is easily contaminated.

NASBA is expanded upon in Compton J. "Nucleic acid sequence-based amplification" (1991) Nature 350:91-92; Heim AI et al "Highly sensitive detection of gene expression of an intronless gene; amplification of mRNA) but not genomic DNA by nucleic acid sequence based amplification (NASBA)" (1998) Nucleic Acids Research 26(9):2250-2251; and Deiman B et al "Characteristics and applications of nucleic acid sequence based amplification (NASBA)" (2002) Molecular Biotechnology 20:163-179.

A highly automated method termed real-time PCR (which Will be referred to throughout as RT-PCR) has proved to be a highly specific and user-friendly technology but occasionally may have a lower sensitivity than nested PCR. RT-PCR incorporates the use of an additional DNA oligonucleotide molecule labeled at one end with a fluorescence absorbing moiety and at the other end by a fluorescence emitting moiety, which hybridizes to a region of the target gene between the DNA oligonucleotide primers. The labeled molecule is known as a fluorogenic probe. As the amplification process continues the fluorescence signal increases due to the displacement and degradation of the fluorogenic probe by the advancing DNA polymerase enzyme. Real-time PCR is expanded on in Desjardin LE et al "Comparison of the ABI 7700 system (TaqMan) and competitive PCR for quantification of IS6110 DNA in sputum during treatment of tuberculosis." (1998) J Clin Microbiol. 36(7): 1964-8 and Wang T et al "mRNA quantification by real time TaqMan polymerase chain reaction: validation and comparison with RNase protection" (1999) Anal Biochem. 269(1); 198-201.

Yang at al "Clinical detection of polymerase gene of SARS-associated coronovirus" Academic J First Med (2003) 23(5): 424-427, punished on 8 May 2003, is directed to the use of reverse transcriptase PCR using SARS coronavirus-specific primers. Drosten et al, mentioned previously, is directed to the separate use of nested RT-PCR or RT-PCR for the detection of the STARS coronavirus.

Conventional PCR, nested PR and RT-PCR alone may not be sufficiently accurate or sensitive to detect the SARS coronavirus in a wide range of patient samples. Negative results by PCR do not necessarily mean that the patient does not have SARS, as many factors affect viral nucleic acid detection, including insensitivity of the primers and probes used in the amplification protocol, the absence of SARS-CoV infection, the observed illness being caused by another infectious agent, false negative PCR results, or the specimens were collected at a time when the virus or its genetic material was not present or was at an undetectable level with the current methods. Therefore, it is important to devise a method that is sufficiently sensitive to screen effectively for SARS-CoV and to improve the diagnosis of SARS by detecting a specific portion of the nucleic acid of the SARS coronavirus by an amplification technique that combines high sensitivity, high specificity and speed.

It is to be understood that this invention is not limited to the detection of the SARS coronavirus only and is applicable to nucleic acid detection in general. Thus, this invention is applicable to the detection of many diffèrent types of pathogens and viruses, including the SARS coronavirus.

It is ari object of this invention to provide a highly sensitive method for detecting nucleic acids, including the SARS coronavirus, such that a person in the early Stages of infection can be idenfified.

It is also an object of this invention to design a user-friendly diagnostic kit to detect the nucleic acid

### Summary of the invention

In general, the present invention provides a process for the extraction and detection of, for example, a coronavirus nucleic acid from various biological or environmental samples, which comprises the following steps (A) Collecting a sample that is suspected to contain the STARS coronavirus (B) Extracting the ribonucleic acid (RNA) from the collocted sample (C) Amplifying specific target sequence using RNA/DNA amplification technology and (D) Detecting the target amplified nucleic acid products using DNA. amplification technology and a suitable reporting system such as fluorogenic probes, including Taqman^{®} probes or molecular beacons,

Furthermore, the present invention also provides a kit for detecting SARS coronavirus that includes a nucleic acid replicating agent for replicating a target molecule, wherein the target molecule includes a nucleic acid sequences containing at least a portion complementary to the RNA sequence of the SARS coronavirus; and a nucleic acid replicating and detecting agent for amplifying the replicated target molecule and detecting the replicated product during amplification. These agents include a primer set that can amplify the replicated product and a fluorogenic probe that is complementary to the amplified product.

According to a first aspect of the invention, there is provided an isolated DNA sequence comprising any of SEQ ID Nos 1 to 27.

According to a second aspect there is provided an isolated DNA sequence comprising any of SEQ ID Nos 1 to 27 for use in DNA amplification as a primer or as a fluorescently labeled probe. The primer or probe may consist of any of SEQ ID Nos 1 to 27.

According to a third aspect of the invention, there is provided a method for nucleic acid detection comprising the steps of nucleic acid isolation followed by DNA amplification and subsequently Real Time PCR. Preferably, DNA amplification comprises PCR, NASBA or any other suitable nucleic acid amplification technique.

Most preferably, the method comprises the following steps:
i) extracting the nucleic acid from a biological or environmental sample; and
ii) amplifying the nucleic acid, optionally using PCR, NASBA or any other nucleic acid amplification technique ; and
iii) amplifying and detecting the nucleic acid produced in step (ii) using Real Time PCR.

More preferably, step (ii) comprises amplifying the nucleic acid using PCR. Step (ii) is also known as the pre-amplification step, This amplification step may comprise DNA amplification and detection by PCR. Alternatively, NASBA may be used. The other suitable nucleic acid amplification techniques of step (ii) may be chosen from polymerase chain reaction (PCR) followed by agarose, gel electrophoresis, nested PCR, real-time Taqman^{®} PCR, molecular beacon PCR, isothermal and chimeric primer-initiated amplification of nucleic acids (ICAN).

The method of the present invention is achieved by this novel combination of nucleic acid amplification techniques. The combination of pre-amplification step followed by Real Time PCR advantageously provides a highly sensitive method for detecting nucleic acids. This ensures that a person in the early stages of infection by a virus or other pathogen can readily be identified.

Still further aspects of the invention relate to isolated DNA sequence comprising SEQ ID Nos 1 to 27 for use in a method of detecting a nucleic acid and the use of the isolated DNA sequences comprising SEQ ID Nos 1 to 27 in the manufacture of a composition for use in a method of detection of nucleic acid comprising the steps:
i) extracting the nucleic acid from a biological or environmental sample; and
ii) amplifying the nucleic acid, optionally by PCR, NASBA or any other nucleic acid amplification technique; and
iii) amplifying and defecting the nucleic acid from step (ii) using Real Time PCR.

Step (ii) is also known as the pro-amplification step, Pro-amplification may be carried out by PCR or NASBA. The primer used for this pre-amplification step will differ depending on which method is used.

The nucleic acid to be detected is preferably derived from SARS coronavirus RNA or cDNA.

The present invention is directed in general to a diagnostic test kit for detecting SARS coronavirus in a biological or environmental sample comprising:
(i) an isolating agent for isolating the SARS coronavirus RNA from the sample; and
(ii) a nucleic acid replicating agent for replicating a target molecule, wherein the target molecule includes: a nucleic acid sequence complementary to at least a portion of the RNA sequence of SARS coronavirus; and
(iii) a nucleic acid detecting agent for detecting the target molecule, wherein the nucleic acid detecting agent includes the detection molecule.

A still further aspect of the invention relates to a SARS diagnostic test kit comprising two or more isolated DNA sequences corresponding to SEQ ID Nos. 1 to 27.

Preferably, the SARS diagnostic test kit comprises pre-amplification primers corresponding to any one of SEQ ID Nos 1,2, 3, 4 or 5 and 6, 7, 8, 9 or 10, One primer of the primer pair is chosen from SEQ ID Nos 1, 2, 3, 4 or 5 and the other primer is chosen from SEQ ID Nos 6, 7, 8, 9 or 10. SEQ ID Nos 1 to 5 corresponds to PCR forward primers and SEQ ID NOS 6 to 10 correspond to PCR reverse primers. Alternatively, the pre-amplification primers correspond to SEQ ID Nos 26 and 27. SEQ ID Nos 1,2, 3, 4 or 5 and 6, 7,8,9 or 10 may be used in pre-amplification PCR and SEQ ID Nos 26 and 27 may be used in pre-amplification NASBA.

Additionally, the SARS diagnostic test kit also comprises Real Time PCR primers corresponding to any one of SEQ ID NOS 11, 12, 13, 14 or 15 and 16, 17, 18, 19 or 20. One primer of the RT-PCR primer pair is chosen from SEQ ID Nos 11, 12, 13, 14 or 15 and the other primer is chosen from SEQ ID Nos 16,17,18,19 or 20. Furthermore, the SARS diagnostic test kit may also comprise Real Time PCR probes corresponding to any one of SEQ ID Nos 21,22,23,24 or 25. These primers and probes are used in Real Time PCR. SEQ ID Nos 11 to 15 corresponds to Real Time PCR forward primers and SEQ ID NOS 16 to 20 corresponds to Real Time PCR reverse primers. SEQ ID Nos 20 to 25 correspond to Real Time PCR probes.

The primers used in pre-amplification PCR and RT-PCT may be used interchangeably if necessary.

Alternatively, the diagnostic test kit comprises the following primer sels a) SEQ ID Nos 1, 2 or 3 and (6, 7 or 8 and b) SEQ ID Nos 11, 12 or 13 and 16, 17 or 18 and a probe selected from SEQ ID Nos 21, 22 or 23.

As a preferred embodiment) the diagnostic test kit comprises the following primer sets A and B; and C and D; and a probe E:
A) a primer selected from any one of SEQ ID NOS 1, 2, 3, 4 or 5; and
B) a primer selected from any one of SEQ ID NOS 6, 7 , 8 , 9 or 10; and
C) a primer selected from any one of SEQ ID NOS 11, 12, 13, 14 or 15; and
D) a primer selected from any one of SEQ ID NOS 16, 17, 18, 19 or 20; and
E) a probe selected from any one of SEQ ID NOS 20 to 25.

This diagnostic test kit can be used when the pre-amplification step involves PCR. Additionally, primer sets A and B; or C and D may be used interchangeably for either conventional PCR and/or RT/PCR.

As an alternative preferred embodiment, the diagnostic test kit comprises the following primer sets A; and C and D and a probe E:
A) a primer selected from SEQ ID NOS 26 or 27; and
C) a primer selected from SEQ ID NOS 11, 12, 13, 14 or 15; and
D) a primer selected from SEQ ID NOS 16, 17, 18, 19 or 20; and
E) a probe selected from any one of SEQ ID NOS 20 to 25.
This diagnostic test kit can be used when the pre-amplification step involves NASBA. SEQ ID Nos 26 and 27 correspond to SARS NASBA T7 primer and SARS NASBA electrochemiluminensence (ECL) primer respectively,

### Brief description of the drawings

This invention will now be described with reference to the following figures:
Figure 1 shows a flow chart of the overall method of the detection of SARS coronavirus according to a preferred embodiment of this invention.
Figure 2 shows the method for the isolation of SARS coronavirus and its conversion to complementary DNA (cDNA) by a preferred embodiment of this invention.
Figure 3 shows the amplification of a portion of the SARS coronavirus cDNA molecule by two DNA molecules, primets A and B, and the subsequent use of the amplified product as a template for further amplification by two DNA molecules, primers C and D, with detection and quantification effected by probe E according to a preferred embodiment of this invention.
Figure 4 shows the nucleic acid sequences corresponding to SEQ ID Nos.1-27.
Figures 5 to 8 show a comparison of enhanced and standard real-time amplification. For each sample, the ΔRn (the ratio of the amount of reporter dye emission to quenching dye emission) is plotted against the cycle number. The intensity of fluorescence is directly related to the amount of input target DNA. The fewer cycles it takes to reach a detectable level of fluorescence the greater the initial copy number.

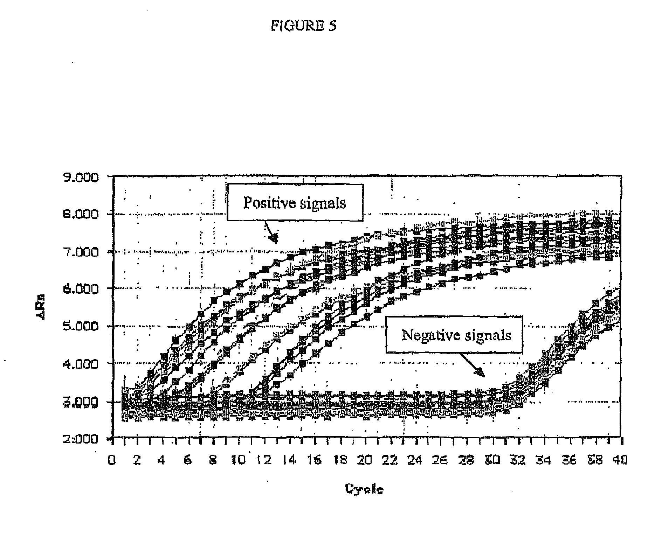

Figure 5 shows clinical samples analyzed using enhanced real-time PCR. The SARS-CoV positive samples are clearly distinguished from the SARS-CoV negative samples, indicated by arrows.

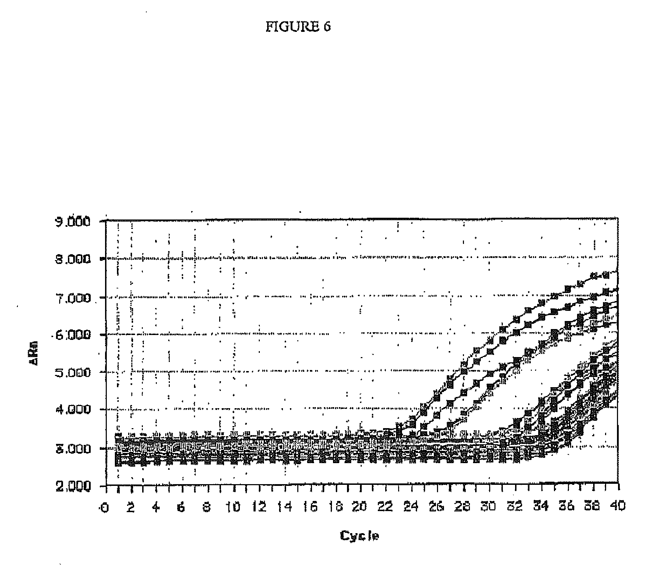

Figure 6 shows the same clinical samples shown in panel A using standard real-time PCR, SARS-CoV, positive and negative samples are not easily differentiated.

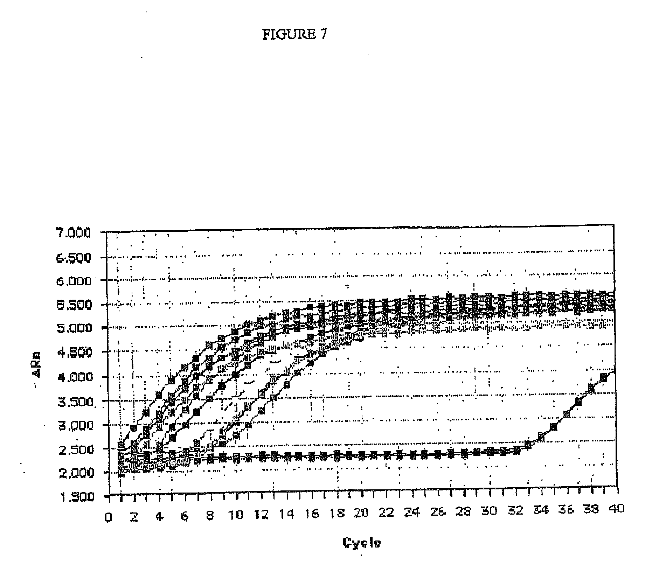

Figure 7 shows serial 10-fold dilutions of SARS-CoV (2 x 10^{2.5} TCID_{50/ml} [uppermost trace] to 2 x 10^{-10.5} TCID₅₀/ml) using enhanced real-time PCR. Lower traces show reverse transcription negative control and TaqMan^{®} negative control.

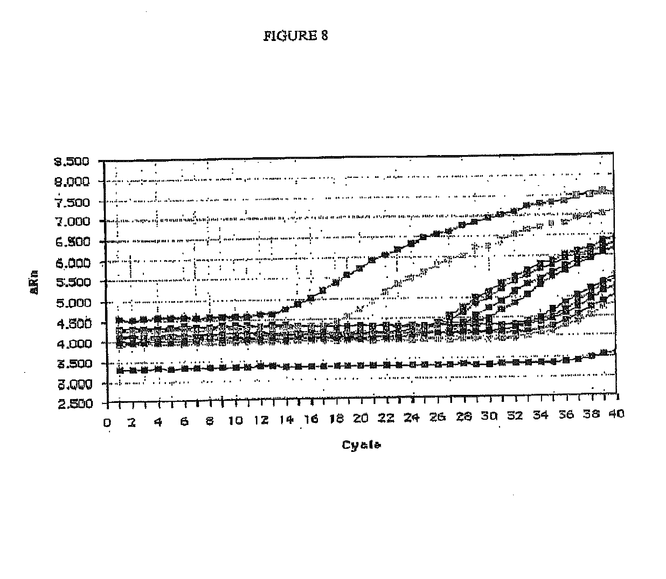

Figure 8 shows serial 10-fold dilutions of SARS-CoV (2 x 10^{2.5} TCID₅₀/ml (uppermost trace 1 to 2 x 10^{-10.5} TCID₅₀/ml) using standard real-time PCR.

### Detailed description of preferred embodiments

Nucleic acid amplification has rapidly become a standard laboratory method to confirm the diagnosis of many diseases by demonstrating directly the presence of an infectious organism in patient tissues. Such methods have become indispensable during the SARS outbreak as their specificity and sensitivity enable confirmation of infection rapidly and allow the effects of anti-viral therapy to be monitored. SARS diagnostic assays that rely upon demonstrating the presence of host antibodies to SARS-CoV have been developed using, for example, ELISA and immunofluorescence. However, such tests often lack the sensitivity of nucleic acid methods and cannot detect newly infected patients, as 7-21 days are required for antibodies to reach a detectable level,

The present invention provides a sensitive method for detecting nucleic acids, such as the SARS coronavirus RNA or DNA in biological and environmental samples. Such biological samples include blood, sputum, serum, plasma, nasopharyngeal swabs, oropharyngeal swabs, urine, stools, other body fluids, and also environmental samples such as water, sewage, waste materials, and so forth.

The method of the present invention for nucleic acid detection involves a pre-amplification step followed by Real-time PCR. The contents of Lau et al "A real-time PCR for SARS-coronavirus incorporating target gene pre-amplification" Biochemical and Biophysical Research Communication (2003) 312:1290-1296 are incorporated by reference. PCR and Real Time PCR (RT PCR) are techniques well known in the art. Real-time polymerase chain reaction (RT-PCR) with fluorescent quantification is a rapid, highly sensitive and highly specific method for the detection of DNA and may be applicable to detection of the SARS coronavirus nucleic acid. Results for this technique alone can be obtained in as little as three hours. RT PCR may use fluorescent quantification but is not limited to fluorescent qunatification only. RT PCR encompasses, for example, the use of an additional DNA oligonucleotide molecule (referred herein as the probe) preferably labelled at one end with a fluorescence absorbing moiety and at the other end by a fluorescence emitting moiety. The additional DNA oligonucleotide hybridizes to a region of the nucleic acid of interest between the DNA oligonucleotide primers. Hence, the signal generator may be a fluorogenic molecule, a molecular beacon or another molecule that can be stimulated to emit photons that can be detected and quantified in a suitable detector.

Nested PCR methods, in which the product of an initial round of amplification is used as a template for subsequent amplification, are well known to increase the fidelity and sensitivity of detection of many target genes. Such pre-amplification methods are considered unnecessary for the various real-time PCR applications on account of their generally superior sensitivity over conventional PCR techniques. However, in situations where the target gene of interest is rare and/or present in a background of other contaminants, pre-amplification in accordance with the present invention was surprisingly found to be advantageous in improving the limit of detection.

The method of the present invention, i.e. pre-amplification followed by Real Time PCR, will be referred to herein as "Enhanced Real-time PCR". Pre-amplification may occur by PCR, NASBA or any other nucleic acid amplification technique. One advantage of this ERT technique is that the detection limit for has surprisingly been found to be up to TCID₅₀ (2×10^{-11.5}) which is 100 fold greater than ordinary TaqMan® Real Time PCR and 10⁸ fold greater than conventional PCR. A further advantage is that the turnaround time for this enhanced real-time PCR detection takes about 5.5 hours. In order to further shorten the time of the whole detection method and increase the sensitivity of the test, the method of combining NASBA technology for pre-amplification with Real-time PCR may also be used. When the pre-amplification step comprised NASBA, the turnaround time was advantageously found to be in the region of 4 hours. Thus, the advantage in terms of detection limits and time for this combined pre-amplification/RT-PCR technique were both unexpected and advantageous.

ERT additionally allows the possibility to discriminate between positive and negative samples in a clear manner. This is particularly important in clinical diagnosis which does not allow any mis-diagnosis or interpretation.

The discrimination between positive and negative signals is more ambiguous when the same samples are analysed by standard real time PCR, where the SA RS CoV positive and negative amplification curves tend to overlap closely with on another (Figure 6). Therefore, a clinician less experienced in interpreting real-time PCR results can distinguish easily between positive and negative signals with the enhanced results obtained by ERT. This is a very important advantage of the present invention over conventional real time PCR.

The increased sensitivity of enhanced real-time PCR makes it applicable to many areas beyond simple clinical diagnosis. Given that fomites and sewage have been linked to SARS outbreaks, the suitability of applying nucleic acid detection methods to environmnental samples is obvious. Examining surfaces in public areas (lifts, waiting areas, etc) for evidence of SARS-CoV nucleic acid will provide an opportunity to monitor the transmission of the disease and also to monitor the effectiveness of cleaning/sterilization procedures.

As the signs and symptoms of SARS are highly variable, especially in patients at high-risk such as the elderly where fever, for example, is often not present, a sensitive method for determining the presence of SARS-CoV would be very useful to limit the spread of the virus in clinical settings. Early detection would also enable emergency plans to be implemented sooner to contain local outbreaks and prevent the international spread of SARS-CoV, limiting public concern and potential economic effects.

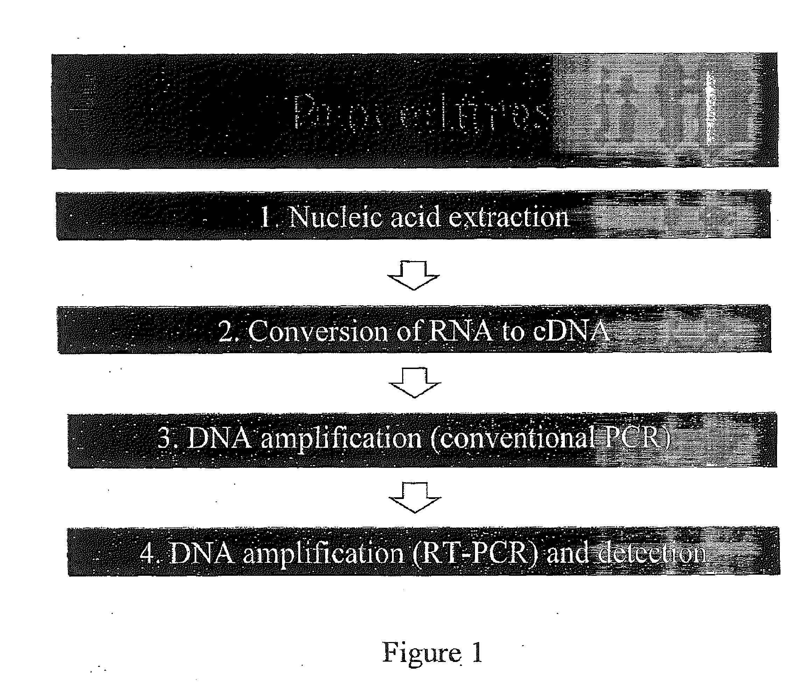

Preferred embodiments of this invention are now described with reference to the Figures. Table 1 contains the reference numerals used in Figures 1 to 3.

**Table 1 : Reference Numerals for Figures 1 to 3.**

| **Reference Numbers** | **Item** |
|---|---|
| 10 | Single-stranded SARS coronavirus RNA |
| 12 | Chromatography column |
| 14 | Mixture of isolated nucleic acids |
| 16 | Complementary DNA (cDNA) |
| 18 | Primer A |
| 20 | Primer B |
| 22 | Extended primer A |
| 24 | Extended primer B |
| 26 | Excess double-stranded product |
| 28 | Primer C |
| 30 | Primer D |
| 32 | Probe E |
| 34 | Excess double-stranded product |

The concentration of SARS coronavirus in a biological sample, for example an oropharyngeal swab, may be very low such that detection of the presence of SARS coronavirus viral RNA may not be performed on the biological sample directly. In order to increase the number of the viral RNA molecules to a sufficient amount for detection purpose, a suitable amplification technology is required. The polymerase chain reaction (PCR) is known to be a flexible technology with particular use for the amplification of DNA. Conversion of RNA into DNA allows the PCR process to be extended to cover pathogens with a RNA genome, such as the SARS coronavirus. The amplified DNA molecules may then be detected by any suitable technology,

The SARS coronavirus contains its genetic material in the form of a single strand of ribonucleic acid (RNA, 10). The viral RNA contains the genes necessary for its reproduction and one of the essential genes is called polymerise. This gene is approximately 2800 nucleotides in length, with the nucleotides numbered from the 5' end of the molecule.

Figure 1 shows the overall procedures for the detection of SARS coronavirus by the detection kit. As shown in Figure 1, the target SARS coronavirus viral nucleic acid molecules, which are in the form of a single strand of RNA, are firstly extracted from a biological sample. The compatible biological sample types may include blood, serum/plasma; peripheral blood mononuclear cells/peripheral blood lymphocytes (PBMC/PBL), sputum, urine, faeces, throat swabs, dermal lesion swabs, cerebrospinal fluids, cervical smears, pus samples, food matrices, and tissues from various parts of the body including brain, spleen, and liver. Other samples that have not been listed may also be used. An isolating agent accomplishes the nucleic acid extraction process of the detection kit of this invention.

After the target SARS coronavirus viral RNA molecules (10) are extracted from the biological sample the amount of RNA molecules in the sample may not be sufficient to be detected. Therefore, a portion of the SARS coronavirus viral RNA molecule is replicated to a target nucleic acid molecule by an appropriate amplification technique, for example, polymerase chain reaction (PCR) or NASBA or RT-PCR. The target nucleic acid molecules may then be detected by suitable methods. The use of a pre-amplification step prior to RT-PCR surprisingly provides a range of benefits including greater detection levels, improved sensitivity and quicker times than provided by conventional PCR techniques alone.

The details of the isolation and amplification procedure will now be discussed.

### Nucleic Acid Isolation:

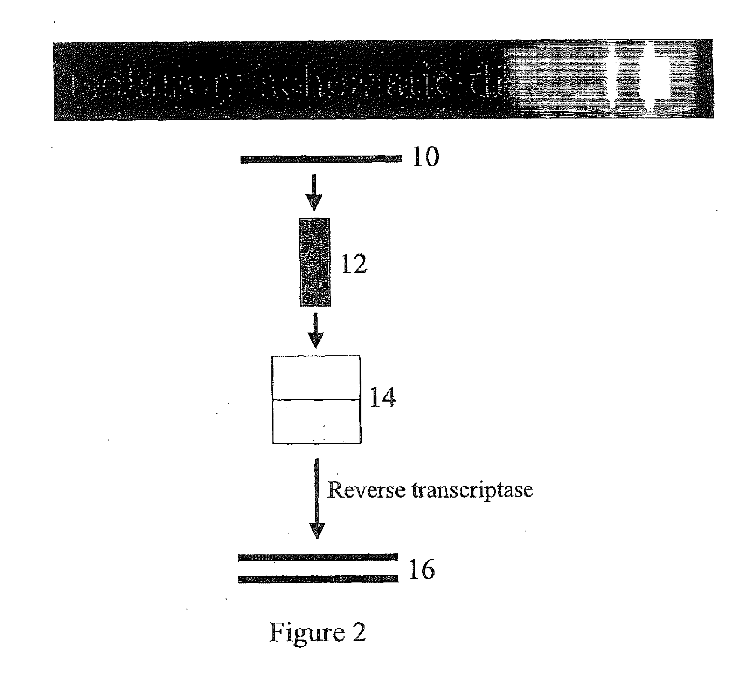

The SARS coranavirus viral RNA molecules (10) may be isolated from the biological sample by applying a suitable isolating agent to the biological sample, Preferably, a lysis agent may be applied before the isolating agent. The lysis agent, for example, a lysis buffer, is responsible for dissolving the proteins and lipids, and denaturing the proteins in the biological sample such that these materials may be removed from the sample more easily. Furthermore, the lysis agent may also serve as a buffer for stabilising the RNA molecule for long-term storage purposes. As shown in Figure 2, the RNA, molecule may be stable in the lysis buffer for up to 48 hours at room temperature and may be stored indefinitely at -70°C. The advantage for doing so is that it may not be necessary to perform the analysis at the sampling site, which may not be suitable for carrying out such processes.

An example of suitable lysis buffers may include 5M guanidine thiocyanate and Tris/HCl. Such lysis buffers are well known in the art. Lysis agents having different composition that can still achieve the purpose of dissolving proteins and lipids, denaturing proteins, and stabilising the RNA molecules may be utilised in the detection kit of this invention.

After the lysis agent has been applied to the biological sample, the next step is the isolation of the nucleic acid molecules from the sample through the use of an isolating agent. Figure 2 describes the overall isolation procedure in the detection kit. After the lysis agent is applied to the biological sample, nucleic acids together with other unwanted components are in the form of a solution. This solution is then applied to a small chromatography column (12) that has a. high affinity for nucleic acid. The non-nucleic acid components are removed from the column by washing. The nucleic acids can then be specifically eluted from the column with a suitable eluent. The nucleic acid purification process described here is well known in the art.

After the nucleic acids contained in the sample are isolated, if necessary, an amplification agent may then be applied to the mixture of nucleic acids (14) such that the SARS coronavirus viral RNA molecules are converted to complementary DNA (cDNA) (16) copies by use of the enzyme reverse transcriptase. This process is well known in the art. Conversion to cDNA is only required in certain situations. Thus, RNA or cDNA can be the template for the next step depending on what procedure is used for pre-amplification.

### Pre-Amplification Step:

A portion of the SARS coronavirus genome is replicated for detection purposes, for example by the PCR technique or NASBA or any other suitable nucleic acid amplification technique, in the so-called pre-amplification step. During the amplification, both cDNA and single stranded RNA are synthesized.

NASBA (nucleic acid sequence-based amplification) is a continuous, isothermal, enzyme-based method for the amplification of nucleic acid. The technique employs a mixture of reverse transcriptase, ribonuclease-H, RNA polymerase and two specially designed DNA oligonucleotide primers. NASBA can work directly on RNA so there is no need to convert DNA to cDNA. SEQ ID NOS 26 and 27 may be used as primers in NASBA pre-amplification step.

Alternatively, conventional PCR or any other nucleic acid amplifcation technique may be used in this pre-amplification step. SEQ ID NOS 1 to 10 may bc used as primers in pre-amplification PCR step. Alternatively, SEQ ID NOS 11 to 20 may be used as primers for this pre-amplification PCR step. For PCR, the SARS RNA is firstly converted to cDNA which is then used as a template for the pre-amplication PCR.

### Pre-Amplification Step by PCR:

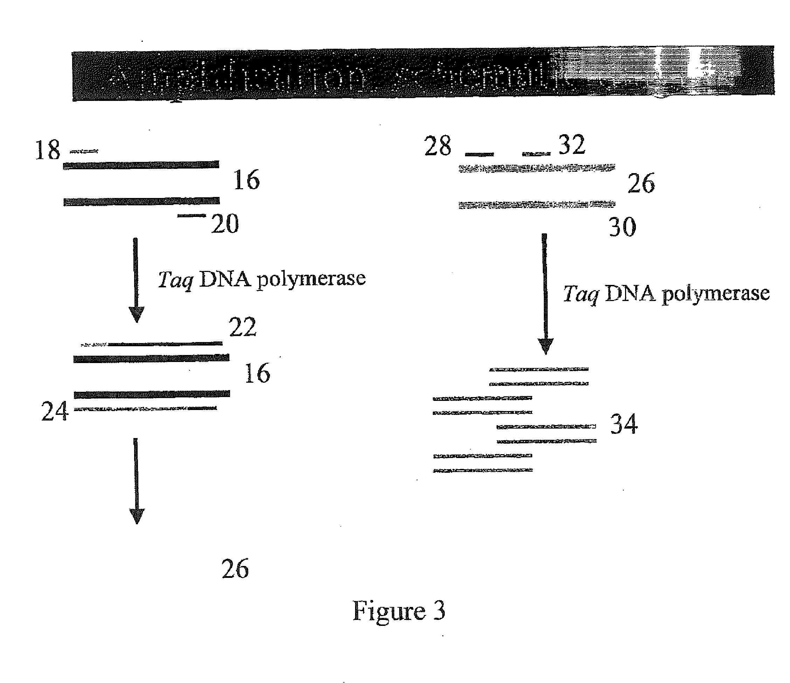

As shown in the Figure 3, the amplification process is initiated by the annealing of primers A (18) and B (20) to the target SARS coronavirus viral cDNA (16), which has been rendered into a single-stranded form by heating to 95°C. The primers A and B are designed such that they are capable of binding to the target cDNA molecule. The precise location of binding depends upon the strain of virus examined. The binding site may change after a certain period of time. The important technical feature of primers A and B is that they remain capable of binding to a portion of the SARS coronavirus viral cDNA, respectively,

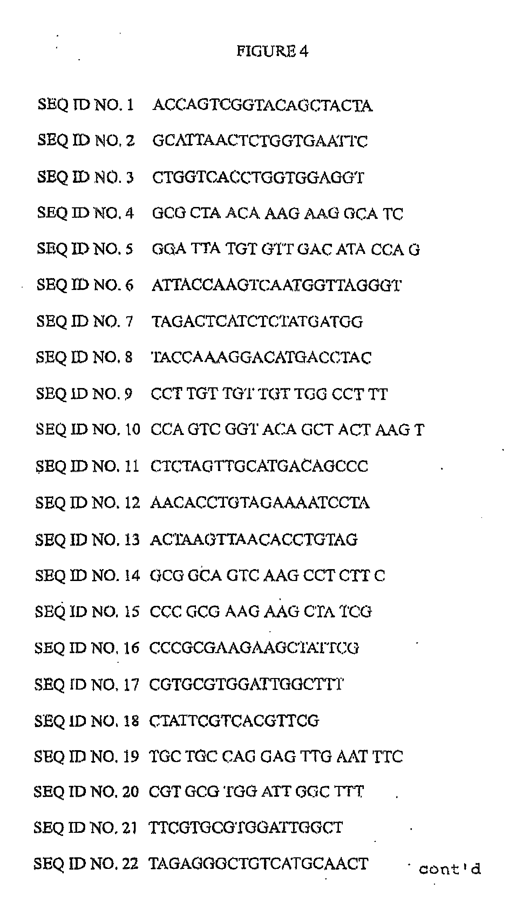

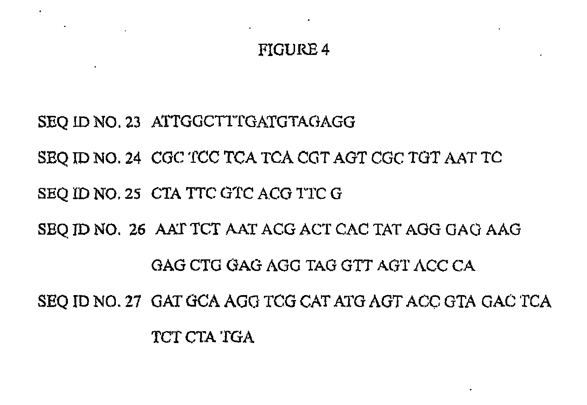

Accordingly, primers A and B include a binding sequence encoding a DNA sequence complementary to at least a portion of SARS coronavirus viral cDNA. For the purpose of this invention, it is found that the region suitable for the binding of primers A to the SARS coronavirus viral cDNA is a region between nucleotides 18319 to 18338 of the polymerase gene of the SARS coronavirus, which is found to contain the least number of nucleotides for the binding function. The type organism used in these studies is SARS coronavirus HKU-39849, GenBank Accession number AY278491. Therefore, the binding sequence of primer A preferably includes a DNA sequence that is complementary to region between nucleotide 18319 to 18338 of the polymerase gene of the SARS coronavirus, which is set forth in SEQ ID No. 1 in Figure 4. It should be noted that SEQ ID No.1 is formally written in the 5'-3', direction. As a result, the orientation of binding with respect to the viral gene is from "back" to "front".

As an alternative, nucleotides 18363 to 18382 (SEQ ID No.2, Figure 4) or nucleotides 18404 to 18386 (SEQ ID No.3, Figure 4) of the polymerase gene of SARS coronavirus may be used for the binding purpose in primer A. Alternatively, SEQ ID Nos 4 or 5 can be used for the binding purposes in primer A.

For the purpose of this invention, it is found that the region suitable for the binding of primer B to the SARS coronavirus viral cDNA is a region between nucleotides 18157 to 18179 of the polymerase gene of the SARS coronavirus, which is found to contain the least number of nucleotides for the binding function. The type organism used in these studies is SARS coconavirus HKU-39849, GenBank Accession number AY278491. Therefore, the binding sequence of primer B preferably includes a DNA sequence that is complementary to region between nucleotide 18157 to 18179 of the polymerase gene of the SARS coronavirus, which is set forth in SEQ ID No. 6 in Figure 4. It should be noted that SEQ ID No. 6 is formally written in the 5'-3' direction.

As an alternative, nucleotides 18125 to 18144 (SEQ ID No.7, Figure 4) or nucleotides 18103 to 18122 (SEQ ID No.8, Figure 4) of the polymerase gene of SARS coronavirus may be used for the binding purpose in primer B. Alternatively, SEQ ID Nos 9 or 10 can be used for the binding purposes in primer B.

After the primers A and B bind to the SARS coronavirus viral cDNA, the primers A and B are extended through the action of a suitable polymerase, for example *Taq* DNA polymerase, in the presence of suitable nucleotides at the 3' end of primers A and B. Therefore, an extended primer A (22) and B (24) including the following sequence (a) a DNA sequence that is complementary to a portion of SARS coronavirus viral RNA is produced.

By application of a suitable amplification program the SARS coronavirus cDNA can be converted into an excess of double-stranded DNA product (26). A suitable amplification program is:

| | |
|---|---|
| Initial Step | 50°C, 2 min |
| DNA Polymerase Activation | 95°C, 3-5 min |
| 40 Cycles | 95°C, 30 sec |
| | 50°C, 45 sec |
| | 72°C, 15 sec |

### Pre-Amplification by NASBA

Alternatively, pre-amplification may occur by NASBA (nucleic acid sequence-based amplification) which is a continuous, isothermal, enzyme-based method for the amplification of nucleic acid. The technique employs a mixture of reverse transcriptase, ribonuclease-H, RNA polymerase and two specially designed DNA oligonucleotide primers. During the amplication, both cDNA and single stranded RNA are synthesized. Therefore, TaqMan real-time PCR can be performed with the presence of cDNA in the NASBA products. RNA may act as the template for NASBA, thus, there is no need for conversion of the SARS RNA to cDNA in this situation.

Suitable primers to carry out NASBA include SEQ ID Nos 26 and 27 which correspond to SARS NASBA T7 primers and SARS NASBA ECL primer respectively. These NASBA primers may be used instead of PCR primers SEQ ID NOS 1 to 10 in the pre-amplification step.

### Real-time PCR

To enhance the sensitivity of the detection process, the products of the pre-amplification step, i.e. PCR reaction or NASBA, are used in a further amplification process, namely the RT-PCR process. SEQ ID NOS 11 to 20 may be used as primers for carrying out RT-PCR, although primer SEQ ID NOS 1 to 10 may alternatively be used, if necessary. SEQ ID NOS 21 to 25 are used as probes in RT-PCR.

To achieve the detection purpose, the amplified double-stranded DNA product (26) of the pre-amplification step, for example PCR or NASBA, is used as the template for a further amplification utilizing RT-PCR, preferably Taqman^{®} quantitative real-time PCR. Before it can be used as the template for further amplification the replicated DNA must first be diluted in a suitable liquid in order to maximize the efficiency of the subsequent amplification process. Suitable diluents for the replicated DNA molecule include nuclease-free water or buffer. A suitable amplification program for the RT-PCR process is:

| | |
|---|---|
| Initial Step | 50°C, 2 min |
| DNA Polymerase Activation | 95°C, 10 min |
| 50 Cycles | 95°C, 15 sec |
| | 72°C, 1 min |

The degree of amplification can be monitored automatically in suitable equipment, e.g. ABI Prism 7700 genetic detection system (Applied Biosystems).

Amplification by RT-PCR requires two DNA oligonucleotide primers (primer C (28) and primer D (30)) and a fluorogenic probe (E, 32).

For the purpose of this invention, it is found that the region suitable for the binding of primer C to the SARS coronavirus viral cDNA amplification product is a region between nucleotides 18264 to 18245 of the polymerase gene of the SARS coronavirus, which is found to contain the least number of nucleotides for the binding function. The type organism used in these studies is SARS coronavirus HKU-39849, GenBank Accession number AY278491. Therefore, the binding sequence of primer C preferably includes a DNA sequence that is complementary to region between nucleotide 18264 to 18245 of the polymerase gene of the SARS coronavirus, which is set forth in SEQ ID No. 11 in Figure 4 It should be noted that SEQ ID No. 11 is formally written in the 5'-3' direction. As a result, the orientation of binding with respect to the viral gene is from "back" to "front".

As an alternative, nucleotides 18295 to 18314 (SEQ ID No. 12, Figure 4) or nucleotides 18322 to 18304 (SEQ ID No. 13, Figure 4) of the polymerase gene of SARS coronavirus may be used for the binding purpose in primer C. Alternatively, SEQ ID Nos 14 or 15 can be used for the binding purposes in primer C.

For the purpose of this invention, it is found that the region suitable for the binding of primer D to the SARS coronavirus viral cDNA amplification product is a region between nucleotides 18193 to 18211 of the polymerase gene of the SARS coronavirus, which is found to contain the least number of nucleotides for the binding function. The type organism used in these studies is SARS coronavirus HKU-39849, GenBank Accession number AY278491. Therefore, the binding sequence of primer D preferably includes a DNA sequence that is complementary to region between nucleotide 18193 to 18211 of the polymerase gene of the SARS coronavirus, which is set forth in SEQ ID No. 16 in Figure 4. It should be noted that SEQ ID No. 16 is formally written in the 5'-3' direction.

As an alternative, nucleotides 18219 to 18336 (SEQ ID No. 17, Figure 4) or nucleotides 18205 to 18220 (SEQ ID No. 18, Figure 4) of the polymerase gene of SARS coronavirus may be used for the binding purpose in primer D. Alternatively, SEQ ID Nos 19 or 20 can be used for the binding purposes in primer D.

For the purpose of this invention, it is found that the region suitable for the binding of probe E to the SARS coronavirus viral cDNA amplification product is a region between nucleotides 18217 to 18234 of the polymerase gene of the SARS coronavirus, which is found to contain the least number of nucleotides for the binding. function. The type organism used in these studies is SARS coronavirus HKU-39849, GenBank Accession number AY278491. Therefore, the binding sequence of probe E preferably includes a DNA sequence that is complementary to region between nucleotide 18217 to 18234 of the polymerase gene of the SARS coronavirus, which is set forth in SEQ ID No. 21 in Figure 4.

As an alternative, nucleotides 18241 to 18260 (SEQ ID No. 22, Figure 4) or nucleotides 18228 to 18246 (SEQ ID No. 23, Figure 4) of the polymerase gene of SARS coronavirus may be used for the binding purpose in probe E. Alternatively, SEQ ID Nos 24 or 25 can be used for the binding purposes in probe E.

The primers C and D extend from the 3' end via the action of a suitable enzyme, e.g. *Taq* DNA polymerase and added nucleotides to form a double-stranded product (34). During the amplification, the probe E is displaced and degraded by the exonuclease action of the DNA polymerase enzyme. This results in an increase in the flluorescence signal which can be correlated to the degree of amplification and hence to the concentration of amplified product. The products of the RT-PCR amplification process is thus a large quantity of target DNA molecules each containing the following DNA sequences:
(a) a DNA sequence complementary to a portion of the original SARS coronavirus

The sequence of the Taqman^{®} Real Time probe may be complementary to any region of the amplified DNA product whose ends are defined by primers C and D. However, the probe sequence cannot overlap that of the primers C and D, as this would prevent the amplification reaction from occurring.

### Enhanced Real-time PCR

This combined pre-amplification and real-time PCR technique is referred to herein as "Enhanced real-time PCR" (ERT). Figure 3 shows a schematic diagram for the amplification of the SARS coronavirus viral RNA by a combination of PCR and RT-PCR.

The salient feature of this new assay is the use of the amplification product from the pre-amplification step, either for example an initial round of conventional PCR or NASBA, as the template for a second round of RT-PCR. This combined technique increases the sensitivity of SARS-CoV detection. Optionally, the same primers may be used for the initial pre-amplification by conventional PCR and the RT-PCR stages. This additional pre-amplification step improves the sensitivity of detection of SARS CoV in clinical samples.

Table 2 shows how the listed primer sequences correspond to the SARS sequence based on CUHK AG03 AY 345988 GenBank sequence.

**Table 2**

| |
|---|
| **SEQ ID NO 1:** 18323 to 18304 |
| **SEQ ID NO 2:** 18367 to 18348 |
| **SEQ ID NO 3:** 18389 to 18371 |
| **SEQ ID NO 6:** 18142 to 18160 |
| **SEQ ID NO 7:** 18110 to 18129 |
| **SEQ ID NO 8:** 18088 to 19107 |
| **SEQ ID NO 11:** 18249 to 18288 (BNI TM SARS S2) |
| **SEQ ID NO 12:** 18299 to 18280 |
| **SEQ ID NO 13:** 18307 to 18289 |
| **SEQ ID NO 16:** 18178 to 18196 (DNA SA HUF) |
| **SEQ ID NO 17:** 18204 to 18221 (DNA SA HUT) |
| **SEQ ID NO 18:** 18190 to 18205 |
| **SEQ ID NO 21:** 18202 to 18219 |
| **SEQ ID NO 22:** 18226 to 18245 |
| **SEQ ID NO 23:** 18213 to 18231 |

SEQ ID Nos 4, 9, 14, 19, 24 are derived from the SARS nucleocapsid protein (NP) gene and SEQ ID Nos 5, 10, 15, 20 and 25 are derived from the SARS polymerase (Pol) gene.

Furthermore, the present invention also relates to the use of a first and a second purified and isolated DNA molecules in the manufacture of a kit for the detection of SARS coronavirus in a biological or environmental sample, wherein the RNA molecule of SARS coronavirus is isolated from the biological or environmental sample by an isolating agent; a target molecule is replicated by a nucleic acid replicating agent including the first and the second purified and isolated DNA molecules, wherein the target molecule includes: a nucleic acid sequence complementary to at least a portion of the RNA sequence of SARS coronavirus; and a nucleic acid sequence for binding to a detection molecule; the target molecule is detected by a nucleic acid detecting agent, wherein the nucleic acid detecting agent includes the detection molecule.

Preferably, the target molecule is a cDNA molecule, although it may be RNA or other nucleic acid. The first purified and isolated DNA molecule may include a DNA sequence for binding to at least a portion of the RNA sequence of SARS coronavirus such that the first purified and isolated DNA molecule extends in the presence of an enzyme and DNA nucleotides to generate a DNA sequence including a DNA sequence complementary to at least a portion of the RNA sequence of SARS coronavirus when the first purified and isolated DNA molecule binds to at least a portion of the cDNA sequence of SARS coronavirus.

More preferably, the first DNA sequence may encodes either one of the DNA sequences set forth in SEQ ID Nos. 1, 2, 3, 4 or 5 in conjunction with either one of the DNA sequences set forth in SEQ ID Nos. 6, 7, 8, 9 or 10. Alternatively, the first DNA sequence may encode the DNA sequences set forth in SEQ ID Nos 26 and 27.

Additionally, the nucleic acid replicating agent may includes a second purified and isolated DNA molecule including a DNA sequence complementary to at least a portion of the RNA sequence of SARS coronavirus such that the second purified and isolated DNA molecule extends in the presence of an enzyme and DNA nucleotides to generate a DNA sequence including a DNA sequence encoding at least a portion of the RNA sequence of SARS coronavirus when the second purified and isolated DNA molecule binds to a DNA molecule including a DNA sequence complementary to at least a portion of the RNA sequence of SARS coronavirus. More preferably, the DNA sequence encodes either one of the DNA sequences set forth in SEQ ID Nos. 11, 12, 13, 14, or 15 in conjunction with either one of the DNA sequences set forth in SEQ ID Nos. 16, 17 , 18, 19 or 20. The replicated DNA product may be first diluted prior to the further amplification.

The detection molecule may includes a DNA sequence encoding at least a portion of the RNA sequence of SARS coronavirus for binding to the target molecule; and a signal generator

The signal generator may comprise one of the following: fluorogenic molecule, molecular beacon, another molecule that can be stimulated to emit photons that can be detected and quantified in a suitable detector. Preferably, the DNA sequence of the detection molecule encodes any one of the DNA sequences set forth in SEQ ID Nos. 20 to 25.

The present invention is now illustrated by the following non-limiting examples. It should be noted that various changes and modifications can be applied to the following example and processes without departing from the scope of this invention.

### Examples

The components and general protocols used in the following examples are expanded on below:

### Samples

The samples used to isolate nucleic acid were obtained from SARS patients and were provided by hospitals in Beijing, China.

### Box A (Nucleic acid isolation)

Qiagen RNeasy^{®} Mini Kit nucleic acid isolation kit

### Box B (Nucleic acid amplification)

**Mastermix** (0.5 ml) (purchased from Eurogentec)

Contains dNTPs with dUTP (0.2 mM of dATP, dCTP, dGTP and 0.4 mM dUTP) HotStart *Taq* DNA polymerase, MgCl₂, (Uracil-N-glycosylase (UNG), and Passive Reference.

### SARS primer (20 µl)

A DNA oligonucleotide complementary to a region of the polymerase gene of the coronavirus associated with SARS and includes SEQ ID Nos 1 to 20 and 26 and 27.

### SARS probe (10 µl)

A DNA oligonucleotide complementary to a region of the polymerase gene of the coronavirus associated with SARS and labeled with both a fluorescence emitting label and a fluorescence quenching module and includes SEQ ID Nos 21 to 25.

0.2 mM of cach SARS primer plus 0.24 mM TaqMan SARS probe are added into the universal mastermix.

### SARS positive control (10 µl)

A highly purified and non-infectious plasmid containing a portion of the polymerase gene of the coronavirus associated with SARS

### Protocols for preparation of reagents

### Box A (Nucleic acid isolation)

A sufficient number of RNeasy mini-columns (one per sample) as required was prepared. RLT buffer (0.4 ml per sample), RW1 buffer (0.7 ml per sample) and RPE buffer (0.5 ml per sample) (all provided in the RNeasy kit) as are required were prepared and warmed gently to room temperature. 70% ethanol (0.4 ml per sample) and nuclease-free water (50 µl per sample) as were required were prepared and warmed gently to room temperature.

### Box B (Nucleic acid amplification)

Mastermix, SARS primers, SARS probes, purified nucleic acid template (sample) and nuclease-free water as are required were prepared and warmed gently to room temperature.

### Nucleic acid extraction Protocol (Qiagen RNeasy^{®} Mini Kit)

1. 0.4 ml sample and 0.4 ml RLT buffer were mixed together.
2. 0.4 ml 70% ethanol was added into a tube
3. 0.6 ml of the solution mix was added into an RNeasy column. The tube was closed gently and centrifuged for 15s at approximately 10,000 x g. The flow-through was discarded in a 2 ml collection tube. Step 3 was repeated.
4. 0.7 ml RW1 buffer was added to the RNeasy column. The tube was closed gently and centrifuged for 15s at approximately 10,000 X g to wash the column. The flow-through was discarded.
5. 0.5 ml RPE buffer was added to the RNeasy column and centrifuged for 15s at approximately 10,000 x g. The flow-through was discarded. Step 5 was repeated.
6. The column was centrifuged for extra 2 min at approximately 10,000 × g
7. The RNeasy column was transferred to a new 1.5 ml collection tube, 50 µl RNase-free water was pipetted directly onto the silica-gel membrane and incubated at RT for 3 min
8. The RNA was eluted by centrifugation for 1 min at approximately 10,000 × g
9. The nucleic acid, RNA, was obtained.

### Sequencing Protocol

The products of the amplification reactions were sequenced to ensure that the amplicons corresponded to the intended target sequence. Sequencing reactions were performed using a commercially available sequencing kit (Applied Biosystems) according to the manufacturer's instruction. DNA sequences were obtained by resolving the sequencing reactions with the 373A sequencer system (Applied Biosystems). DNA sequences were analyzed using the BLAST sequence comparison tool available from the National Center for Biotechnology Information (NCBI) (http://www.ncbi.nlm.nih.gov).

### Example 1:

### Enhanced Real-time PCR using PCR for pre-ampliflcation

### PCR Pre-Amplification:

In a total volume of 25 µl, the following cornponents were mixed; nuclease-free water, 0.2 mM dNTPs, IU Platinum *Taq* DNA polymerase (Invitrogen), 5 mM MgCl₂, 1x PCR buffer, primers (10 µM) and cDNA template (approx. 100 ng). The PCR amplification was performed in a MasterCycler (Eppendorf) with the following temperature profile:

| | |
|---|---|
| Initial Step | 50°C, 2 min |
| DNA Polymerase Activation | 95°C, 3-5 min |
| 40 Cycles | 95°C, 30 sec |
| | 50°C, 45 sec |
| | 72°C, 15 sec |

### Real-time PCR

Following the first round of PCR pre-amplification, a portion of the product is used as template for further amplification utilising RT-PCR.

The PCR components were mixed in the proportions descrihed in Table 3.

**Table 3**

| **Component** | **Volume (µl)** |
|---|---|
| Nuclease-free water | 7.8 |
| Mastermix | 10 |
| SARS Primer | 0.8 |
| SARS Probe | 0.4 |
| Template (100 ng) | 1 |
| | |
| Total Volume | 20 |

The RT-PCR equipment (ABI 7700) was programmed with the amplification profile of Table 4.

**Table 4 : Real-time PCR Profile (ABI Prism 7700 Sequence Detector)**

| | |
|---|---|
| Initial Step | 50°C, 2 min |
| DNA Polymerase Activation | 95°C, 10 min |
| 50 Cycles | 95°C, 15 sec |
| | 72°C, 1min |

The results of SARS coronavirus detection using PCR pre-amplification followed by RT-PCR are shown in Tables 5 and 6. The results were confirmed by DNA sequencing using a Perkin Elmer ABI 310 Genetic Analyzer and the general protocol given above.

### Comparison of different amplification methods on nucleic acid samples obtained from SARS patients

The results shown in Tables 5 and 6 were obtained by attempting to amplify nucleic acid isolated from various patient samples. The samples were obtained from SARS patients and were provided by hospitals in Beijing, China. Each sample was tested by three different methods:
i) conventional PCR with agarose gel electrophoresis,
ii) real-time Taqman^{®} PCR, and
iii) the newly described method, enhanced RT-PCR using a pre-amplification step

Conventional PCR was carried out using the same protocol as the PCR pre-amplification. Real-time Taqman^{®} PCR was carried out using the same protocol as in the ERT method.

**Table 5: Results of different amplification and detection technologies**

| **Sample number** | **Type of Sample** | **Conventional PCR** | **Real Time PCR** | **Enhanced RT-PCR** | **Clinical Diagnosis** |
|---|---|---|---|---|---|
| 1 | White Blood Cell | NEG | NEG | POS | SARS |
| 2 | Nasal swab | NEG | NEG | POS | SARS |
| 3 | Throat swab | NEG | NEG | POS | SARS |
| 4 | Throat swab | NEG | NEG | POS | SARS |
| 5 | Serum | NEG | NEG | POS | SARS |
| 6 | Nasal throat | NEG | NEG | POS | SARS |
| 7 | Stool | NEG | NEG | POS | SARS |
| 8 | Serum | NEG | NEG | POS | SARS |
| 9 | Throat swab | NEG | NEG | POS | SARS |
| 10 | Urine | NEG | NEG | POS | SARS |
| 11 | Urine | NEG | NEG | POS | SARS |
| 12 | Nasal swab | NEG | NEG | POS | SARS |
| 13 | Stool | NEG | NEG | POS | SARS |
| 14 | Serum | NEG | POS | POS | SARS |
| 15 | Urine | POS | NEG | POS | SARS |

### Result of the sensitivity study

Coronavirus RNA was extracted and transcribed into cDNA. The cDNA was serially diluted into and tested by the three methods described in Table 6.

| **Sample Number** | **Dilution** | **Conventional PCR** | **Real Time PCR** | **Enhanced RT-PCR** |
|---|---|---|---|---|
| 1 | 10e-1 | POS | POS | POS |
| 2 | 10e-2 | POS | POS | POS |
| 3 | 10e-3 | POS | POS | POS |
| 4 | 10e-4 | POS | POS | POS |
| 5 | 10e-5 | NEG | POS | POS |
| 6 | 10e-6 | NEG | POS | POS |
| 7 | 10e-7 | NEG | NEG | POS |
| 8 | 10e-8 | NEG | NEG | POS |
| 9 | 10e-10 | NEG | NEG | POS |
| 10 | 10e-11 | NEG | NEG | NEG |
| 11 | 10e-12 | NEG | NEG | NEG |

### Table 6: Sensitivity study

### Example 2

### Enhanced Real-time PCR using PCR for pre-amplification

Clinical samples were collected from 120 patients diagnosed as suspected or probable SARS cases and analyzed by conventional PCR followed by agarose gel electrophoresis, conventional TaqMan^{®} real-time PCR and enhanced Taqman^{®} real-time PCR assays according to the present invention using the protocols of example 1.

Using ERT, An approximately 190 bp region of the SARS-CoV polymerase gene was amplified from the cDNA by conventional PCR. The amplicon was sequenced and confirmed to correspond to the expected region of SARS-CoV. This PCR product was subsequently used as the template for the enhanced real-time PCR protocol using the RT-PCR primers. With this protocol, 28/120 (23.3%) patient samples were identified as SARS-CoV positive compared with 21/120 (18,3%) with the standard real-time method and an average of only 10.6% with the various conventional PCR methods. Suitable negative, positive and PCR inhibition controls were used at each stage of the amplification procedure.

A selection of standard and enhanced real-time PCR data for clinical samples is shown (Figures 5 to 8). The clear differentiation between SARS-CoV positive and SARS-CoV negative samples is obvious (Fig. 5, indicated by arrows). The discrimination between positive and negative signals is more ambiguous when the same samples arc analyzed by standard real-time PCR, where the SARS-CoV positive and negative amplification curves tend to overlap closely with one another (Fig. 7). It is easier to interpret real-time PCR results so as to distinguish easily between positive and negative signals with the enhanced results obtained by ERT. The enhanced real-time PCR method results in a lowering of the cycle time (Ct) value for SARS-CoV positive samples compared with the conventional real-time PCR method. A Ct value of ≤35.0 is a suitable cut-off for distinguishing unambiguously between samples containing SARS-CoV nucleic acid from those that do not.

### Analytical sensitivity

The limit of detection of each amplification method was determined by serially diluting total nucleic acid extracted from a cultured sample of SARS-CoV (2 x 10^{2.5}TCID₅₀/ml) obtained from parent in Hong Kong. The limit of detection of the conventional PCR, standard real-time PCR and the enhanced real-time PCR methods was equivalent to 2 x 10^{-5.5} TCID₅₀/ml, 2x 10^{-10.5} TCID₅₀/ml (Fig. 1D) and 2 x 10^{-12.5} TCID₅₀/ml (Fig. 1C). respectively. Thus, the enhanced real-time PCR method is at least 10²-fold more sensitive than the standard real-time PCR and 10⁷-fold more sensitive than any of the conventional PCR techniques currently used for molecular detection of SARS-CoV. Detection of SARS-CoV by viral culture may be the least sensitive method where only 3/28 (10.7%) cases detected by enhanced real-time PCR were positive for SARS-CoV by viral culture.

Enhanced real-time PCR method was found in several cases to detect SARS-CoV positive samples that were not confirmed by any other assay. Hence, any new detection method that improves the accuracy of diagnosis is a valuable addition to the panoply of diagnostic tools available to a clinician. Conventional PCR and real-time PCR alone identified fewer SARS-CoV positive cases. Results were confirmed by viral culture in 3/28 cases. The limit of detection of the enhanced real-time PCR method was 10²-fold higher than the standard real-time PCR assay and 10⁷-fold higher than conventional PCR methods. The increased sensitivity of the assay may help control the spread of the disease during future SARS outbreaks.

### Example 3:

### Enhanced Real-time PCR using NASBA for pre-amplification

### (i) NASBA reaction

Content of mix for NASBA reagent (final concentration):

| | |
|---|---|
| SARS primer pairs | 0.2 mM (SEQ ID NOS 26 and 27) |
| Tris-Cl, pH 8,5 | 40 mM |
| MgCl2 | 12 mM |
| KCl | 70 mM |
| DTT | 5 mM |
| DMSO | 10% |
| dNTPs | 1 mM each |
| NTPs | 2 mM each |

Content of mix for NASBA enzyme (final concentration):

| | |
|---|---|
| BSA | 0.1 mg/ml |
| T7 RNA polymerase | 30 Units |
| Reverse transcriptase | 8 Units |
| RNase H | 0.1 Unit |

| | **NASBA Reaction mix:** |
|---|---|
| 5 µl | Sample RNA |
| 0.83 µl | SARS primer mix (10µM) |
| 6.67 µl | Reagent mix |
| 1.33 µl | KCl |
| 1.17 µl | Water |
| 15 µl | Total volume: |

The reaction mix was incubated at 65°C for 5 min then cooled to 41°C for 5 min. Then 5 µl enzyme mix (total 55 µl for 1 enzyme sphere) was added to the reaction mix. After brief mixing, it was cooled to 41°C and incubated for 5 min. The reaction mix was briefly spun and then subjected to 1.5 hours of incubation at 41°C.

### (ii) TaqMan Real-time PCR

Following the first round of NASBA pre-amplification, the NASBA product was used as a template for further amplification utilizing RT-PCR as follows:

| | **TaqMan Reaction mix** |
|---|---|
| 2.0 µl | template |
| 1.6 µl | Primers and probe mix (10 µM) |
| 10 µl | Universal mix (2×) |
| 8.9 µl | DEPC-water |
| 20 µl | Total volume: |

### Thermocycle profile:

50°C for 2 min
95°C for 10 min
40 cycles of: 95°C for 15 sec
58°C for 1 min

### Results of NASBA Enhanced real-time PCR:

The Coronavirus NASBA product was found to be successfully amplified and detected by TaqMan real-time PCR as shown in Tables 7, 8 and 9.

**Table 7 : Detection of NASBA product by electrochemiluminensence (ECL) method**

| **Sample** | **NASBA reading** | **Sample** | **NASBA reading** |
|---|---|---|---|
| ***RS*** | 34574 | *TCID50 10⁻¹¹(2×10^{-8.5})* | 401 |
| *TCID50 10⁻²(2×10^{0.5})* | 992660 | *TCID50 10⁻¹²(2×10^{-9.5})* | 355 |
| *TCID50 10⁻⁴(2×10^{-1.5})* | 1566632 | *TCID50 10⁻¹³(2×10^{-10.5})* | 411 |
| *TCID50 10⁻⁶(2×10^{-3.5})* | 10000001 | *TCID50 10⁻¹⁴(2×10^{-11.5})* | 355 |
| *TCID50 10⁻⁷(2×10^{-4.5})* | 2382494 | *TCID50 10⁻¹⁵(2×10^{-12.5})* | 506 |
| *TCID50 10⁻⁸(2×10^{-5.5})* | 7407 | *NASBA negative* | 352 |
| *TCID50 10⁻⁹(2×10^{-6.5})* | 415 | *Negative* | 142 |
| *TCID50 10⁻¹⁰(2×10^{-7.5})* | 341 | | |

**Table 8 : NASBA-TaqMan Real-time PCR**

| **Sample** | **Ct value** | **Sample** | **Ct value** |
|---|---|---|---|
| *TCID50 10⁻²(2×10^{0.5})* | 11.29 | *TCID50 10⁻¹¹(2×10^{-8.5})* | 40 |
| *TCID50 10⁻⁴(2×10^{-1.5})* | 14.12 | *TCID50 10⁻¹²(2×10^{-9.5})* | 40 |
| *TCID50 10⁻⁶(2×10^{-3.5})* | 10.79 | *TCID50 10⁻¹³(2×10^{-10.5})* | 40 |
| *TCID50 10⁻⁷(2×10^{-4.5})* | 8.30 | *TCID50 10⁻¹⁴(2×10^{-11.5})* | 40 |
| *TCID50 10⁻⁸(2×10^{-5.5})* | 7.16 | *TCID50 10⁻¹⁵(2×10^{-12.5})* | 40 |
| *TCID50 10⁻⁹(2×10^{-6.5})* | 40 | *Negative* | 40 |
| *TCID50 10⁻¹⁰(2×10^{-7.5})* | 40 | | |

**Table 9 : Conventional Real-time PCR**

| **Sample** | **Ct value** | **Sample** | **Ct value** |
|---|---|---|---|
| *TCID50 10⁻³(2×10^{-0.5})* | 25.99 | *TCID50 10⁻¹⁰(2×10^{-7.5})* | 40 |
| *TCID50 10⁻⁴(2×10^{-1.5})* | 32.82 | *TCID50 10⁻¹¹(2×10^{-8.5})* | 40 |
| *TCID50 10⁻⁵(2×10^{-2.5})* | 40 | *TCID50 10⁻¹²(2×10^{-9.5})* | 40 |
| *TCID50 10⁻⁶(2×10^{-3.5})* | 40 | *TCID50 10⁻¹³(2×10^{-10.5})* | 40 |
| *TCID50 10⁻⁷(2×10^{-4.5})* | 40 | *TCID50 10⁻¹⁴(2×10^{-11.5})* | 40 |
| *TCID50 10⁻⁸(2×10^{-5.5})* | 40 | *TCID50 10⁻¹⁵(2×10^{-12.5})* | 40 |
| *TCID50 10⁻⁹(2×10^{-6.5})* | 40 | *Negative* | 40 |

The reaction conditions for NASBA-Taqman PCR were found to be optimized. The sensitivity of NASBA-TaqMan and ordinary NASBA is similar which is approximately TCID50 2x 10^{-5.5}. From table 7 (NASBA ECL result), it is shown that the test can detect down to TCID 50 x 10-8, which is the same as NASBA Taqman PCR (ERT). However, the reading of the NASBA ECL is 7407 which is very different from other positive signals (usually over million counts). Therefore, in a real situation, this result cannot be easily judged as positive. For NASBA Real time PCR (ERT), however, the Ct is 7.16 which is similar to other positive signals. Therefore, our NASBA real time PCR (ERT) still has an advantage over conventional NASBA method.

As shown in the above examples, it can be realised that the detection kit may be used conveniently in various testing sites. Furthermore, the detection kit is relatively easier to use than existing methods, and may be able to provide the detection results in a shorter time, the detection results may be available within three hours if desired. As it is a DNA-based detection system, the specificity and the sensitivity may be enhanced, the detection kit described in the examples are specific to the SARS coronavirus, and the concentration of the SARS coronavirus in the sample may no longer be important, as the virus will be replicated to a target molecule for detection.

Although the preferred embodiment of this invention has been described in previous paragraphs, it should be apparent to one skilled in the art that modifications and alternative editions of this invention are possible, and such modifications and editions are still within the scope of this invention, which is set forth in the following claims. In addition, the embodiments of this invention shall not be interpreted restrictively by the examples or figures only.

The invention will now be described by way of a series of numbered clauses.
1. An isolated DNA sequence comprising any of SEQ ID Nos 1 to 27.
2. An isolated DNA sequence of clause 1, consisting of any of SEQ ID Nos 1 to 27.
3. An isolated DNA sequence of clause 1 or 2, for use in DNA amplification.
4. An isolated DNA sequence of clause 3 for use as a primer or a probe, wherein the probe may be fluorescently labeled
5. A method for nucleic acid detection comprising the steps of nucleic acid isolation followed by nucleic acid amplification and subsequently Real Time PCR
6. A method of clause 5 where nucleic acid amplification comprises PCR, NASBA or any other nucleic acid amplification technique.
7. The method for nucleic acid detection of clause 5 or 6 wherein Real Time PCR uses fluorescently labeled probes.
8. The method of any one of clause 5 to 7, wherein the nucleic acid to he detected is DNA or cDNA.
9. The method of clause 8 wherein the nucleic acid is SARS coronavirus cDNA.
10. The method of clause 5 to 9 wherein the method comprises the following steps : i) extracting the nucleic acid from a biological or environmental sample ; amplifying the nucleic acid, optionally using PCR, NASBA or any other nucleic acid amplification technique ; and iii) amplifying and detecting the nucleic acid produced in step (ii) using Real Time PCR.
11. The method of clause 10 wherein there is an additional step after step (i) if the nucleic acid obtained is RNA, wherein the step comprises converting the RNA to cDNA using reverse transcriptase.
12. The method of any one of clauses 5 to 11 wherein primers and/or probes are used in steps (ii) and (iii).
13. The method of clause 12 for the detection of SARS coronavirus cDNA wherein the primers and/or probes bind to at least a portion of SARS coronavirus cDNA.
14. The method of clause 13 wherein the primers and probes used correspond to isolated DNA sequences comprising SEQ ID Nos 1 to 27.
15. The method of clause 14 wherein the primers used in step (iii) do not overlap with the probe.
16. The method of clause 14 wherein the primer sets used in step (ii) corresponds to any of SEQ ID Nos 1, 2, 3, 4 or 5; and 6, 7, 8, 9 or 10.
17. The method of clause 14 wherein the primer set used in step (ii) corresponds to any of SEQ ID Nos 26 and 27.
18. The method in either clause 1, 4 or 15 wherein the primer set used in step (iii) corresponds to any of SEQ ID Nos 11, 12, 13, 14, or 15; and 16,17, 18, 19 or 20.
19. The method in any of clauses 16 to 18 wherein the probe used in step (iii)) corresponds to any of SEQ ID Nos 21, 22, 23, 24 or 25.
20. An isolated DNA sequence of clause 1 for use in a method of detecting a nucleic acid.
21. An isolated DNA sequence for use of clause 20 wherein the nucleic acid is the SARS coronavirus RNA or cDNA.
22. Use of the isolated DNA sequences of clause 1 in the manufacture of a composition for use in a method of detection of nucleic acid comprising the steps: i) extracting the nucleic acid from a biological or environmental sample; and ii) amplifying the nucleic acid, optionally using PCR, NASBA or any other nucleic acid amplification technique; and iii) amplifying and detecting the nucleic acid from step (ii) using Real Time PCR.
23. Use of clause 22 wherein the nucleic acid is DNA or cDNA.
24. Use of clause 23 wherein the nucleic acid is the SARS coronavirus cDNA.
25. Use of a primer specific to SARS coronavirus in the manufacture of a diagnostic test kit for detecting viral infections wherein the virus is SARS coronavirus and the primers consist of SEQ ID Nos 1 to 27.
26. A SARS diagnostic test kit comprising two or more isolated NA sequences corresponding to SEQ ID Nos. 1 to 27.
27. A SARS diagnostic test kit of clause 26 comprising the primers corresponding to any of SEQ ID Nos 1, 2, 3, 4 or 5 and 6, 7, 8, 9 or 10.
28. A SARS diagnostic test kit of clause 26 comprising the primers corresponding to any of SEQ ID Nos 26 and 27.
29. A SARS diagnostic test kit of any of clauses 26 to 28 comprising primers corresponding to any of SEQ ID NOS 11, 12, 13, 14 or 15 and 16, 17, 18, 19 or 20.
30. A SARS diagnostic test kit of clause 29 comprising a probe corresponding to any of SEQ ID Nos 21,22, 23,24 or 25.
31. A diagnostic test kit for detecting SARS coronavirus in a biological or environment sample wherein the kit comprises : (i) an isolating agent for isolating the SARS coronavirus RNA from the sample ; and (ii) a nucleic acid replicating agent for replicating a target molecule, wherein the target molecule includes: a nucleic acid sequence complementary to at least a portion of the RNA sequence of SARS coronavirus ; and (iii) a nucleic acid detecting agent for the target molecule, wherein the nucleic acid detecting agent includes the detection molecule.
32. A kit tor detecting SARS coronavirus of clause 31, wherein the target molecule is a cDNA molecule.
33. A kit for detecting SARS coronavirus of clause 31 or 32, wherein the nucleic acid replicating agent includes a first purified and isolated DNA molecule including : a DNA sequence for binding to at least a portion of the RNA sequence of SARS coronavirus such that the first purified and isolated DNA molecule extends in the presence of an enzyme and DNA nucleotides to generate a. DNA sequence including : a DNA sequence complementary to at least a portion of the cDNA sequence of SARS coronavirus when the first purified and isolated DNA molecule binds to at least a portion of the cDNA sequence of SARS coronavirus.
34. A kit for detecting SARS coronavirus of any of clauses 31 to 33, wherein the first DNA sequence encodes either one of the DNA sequences of SEQ ID Nos. 1,2, 3,4 or 5 in conjunction with either one of the DNA sequences set forth in SEQ ID Nos. 6, 7, 8,9 or 10 or alternatively SEQ ID Nos 26 and 27.
35. A kit for detecting SARS coronavirus of any of clauses 31 to 33, wherein the nucleic acid replicating agent includes a second purified and isolated DNA molecule including: a DNA sequence for binding to at least a portion of the DNA sequence of SARS coronavirus produced by a first round of amplification such that the second purified and isolated DNA molecule extends in the presence of an enzyme and DNA nucleotides to generate a DNA sequence including : a DNA sequence complementary to at least a portion of the cDNA sequence of SARS coronavirus produced during a first round of amplification when the second purified and isolated DNA molecule binds to at least a portion of the cDNA sequence of SARS coronavirus.
36. A kit for detecting SARS coronavirus of any of clauses 32 to 33, wherein the second DNA sequence encodes either one of the DNA sequences set forth in SEQ TD Nos. 11, 12, 13,14 or 15, in conjunction with either one of the DNA sequences set forth in SEQ ID Nos. 16, 17, 18, 19 or 20.
37. A kit for detecting SARS coronavirus of any of clauses clause 31 to 36, wherein the replicated DNA product is first diluted prior to the further amplification.
38. A kit for detecting SARS coronavirus of any one of clauses 31 to 37, wherein the detection molecule includes : a DNA sequence encoding at least a portion complementary to the RNA sequence of SARS coronavirus for binding to the target molecule ; and a signal generator.
39. A kit of clause 38 wherein the DNA sequence of the first DMA molecule does not overlap with the second DNA molecules.
40. A kit of clause 38 or 39 wherein the signal generator comprises one of the following; a fluorogenic molecule, a molecular beacon, or another molecule that can be stimulated to emit photons that can be detected and quantified in a suitable detector.
41. A kit for detecting SARS coronavirus of any of clauses 38 to 40 wherein the detection molecule encodes any one of the DNA sequences of SEQ ID Nos. 21 to 25.

## Claims

**1.** A method for nucleic acid detection comprising the steps of:
(i) extracting the nucleic acid from a biological or environmental sample;
(ii) amplifying the nucleic acid prior to Real Time PCR; and,
(iii) amplifying and detecting the nucleic acid produced in step (ii) using Real Time PCR.

**2.** The method of claim 1, wherein Real Time PCR uses fluorescently labelled probes.

**3.** The method of any one of the preceding claims, wherein the nucleic acid to be detected is DNA or cDNA.

**4.** The method of any one of the preceding claims wherein step (ii) comprises amplifying the nucleic by PCR, NASBA, PCR followed by agarose gel electrophoresis, nested PCR, real-time Taqman PCR, molecular beacon PCR, isothermal or chimeric primer-initiated amplification of nucleic acids (ICAN).

**5.** The method of claim 4, wherein step (ii) comprises amplifying the nucleic acid by NASBA or PCR.

**6.** The method of any one of the preceding claims, wherein step (ii) further comprises nucleic acid detection.

**7.** The method of any one of the preceding claims, further comprising an additional step after step (i) if the nucleic acid obtained is RNA, wherein the step comprises converting the RNA to cDNA using reverse transcriptase,

**8.** The method of any one of the preceding claims, wherein primers and/or probes are used in steps (ii) and (iii).

**10.** The method of claim 9, wherein the primers used in step (iii) do not overlap with the probe.


SEQUENCE LISTING

<110>  HONG KONG DNA CHIPS LIMITED
  YU, Chueng Hoi       LAU, Lok Ting

<120>  Nucleic Acid Detection

<130>  FP5260

<150>  GB 0310181.3

<151>  2003-05-02

<160>  27

<170>  PatentIn version 3.1

<210>  1

<211>  20

<212>  DNA

<213>  SARS Coronavirus

<400>  1
accagtcggt acagctacta                                                    20


<210>  2

<211>  20

<212>  DNA

<213>  SARS Coronavirus

<400>  2
gcattaactc tggtgaattc                                                    20


<210>  3

<211>  19

<212>  DNA

<213>  SARS Coronavirus

<400>  3
ctggtcacct ggtggaggt                                                     19


<210>  4

<211>  20

<212>  DNA

<213>  SARS Coronavirus

<400>  4
gcgctaacaa agaaggcatc                                                    20

<210>  5

<211>  22

<212>  DNA

<213>  SARS Coronavirus

<400>  5
ggattatgtg ttgacatacc ag                                                22


<210>  6

<211>  23

<212>  DNA

<213>  SARS Coronavirus

<400>  6
attaccaagt caatggttag ggt                                               23


<210>  7

<211>  20

<212>  DNA

<213>  SARS Coronavirus

<400>  7
tagactcatc tctatgatgg                                                   20


<210>  8

<211>  20

<212>  DNA

<213>  SARS Coronavirus

<400>  8
taccaaagga catgacctac                                                   20


<210>  9

<211>  20

<212>  DNA

<213>  SARS Coronavirus

<400>  9
ccttgttgtt gttggccttt                                                   20


<210>  10

<211>  22

<212>  DNA

<213>  SARS Coronavirus

<400>  10
ccagtcggta cagctactaa gt                                                22

<210>  11

<211>  20

<212>  DNA

<213>  SARS Coronavirus

<400>  11
ctctagttgc atgacagccc                                                   20

<210>  12

<211>  20

<212>  DNA

<213>  SARS Coronavirus

<400>  12
aacacctgta gaaaatccta                                                   20

<210>  13

<211>  19

<212>  DNA

<213>  SARS Coronavirus

<400>  13
actaagttaa cacctgtag                                                    19

<210>  14

<211>  19

<212>  DNA

<213>  SARS Coronavirus

<400>  14
gcggcagtca agcctcttc                                                    19

<210>  15

<211>  18

<212>  DNA

<213>  SARS Coronavirus

<400>  15
cccgcgaaga agctatcg                                                    18

<210>  16

<211>  19

<212>  DNA

<213>  SARS Coronavirus

<400>  16
cccgcgaaga agctattcg                                                   19

<210>  17

<211>  18

<212>  DNA

<213>  SARS Coronavirus

<400>  17
cgtgcgtgga ttggcttt                                                    18

<210>  18

<211>  16

<212>  DNA

<213>  SARS Coronavirus

<400>  18
ctattcgtca cgttcg                                                      16

<210>  19

<211>  21

<212>  DNA

<213>  SARS Coronavirus

<400>  19
tgctgccagg agttgaattt c                                                21

<210>  20

<211>  18

<212>  DNA

<213>  SARS Coronavirus

<400>  20
cgtgcgtgga ttggcttt                                                    18

<210>  21

<211>  18

<212>  DNA

<213>  SARS Coronavirus

<400>  21
ttcgtgcgtg gattggct                                                    18


<210>  22

<211>  20

<212>  DNA

<213>  SARS Coronavirus

<400>  22
tagagggctg tcatgcaact                                                  20


<210>  23

<211>  19

<212>  DNA

<213>  SARS Coronavirus

<400>  23
attggctttg atgtagagg                                                   19


<210>  24

<211>  29

<212>  DNA

<213>  SARS Coronavirus

<400>  24
cgctcctcat cacgtagtcg ctgtaattc                                        29


<210>  25

<211>  16

<212>  DNA

<213>  SARS Coronavirus

<400>  25
ctattcgtca cgttcg                                                      16


<210>  26

<211>  56

<212>  DNA

<213>  SARS Coronavirus

<400>  26
aattctaata cgactcacta tagggagaag gagctggaga ggtaggttag taccca          56


<210>  27

<211>  42

<212>  DNA

<213>  SARS Coronavirus


<400>  27
gatgcaaggt cgcatatgag taccgtagac tcatctctat ga                         42